# EUROPÄISCHE PATENTSCHRIFT

(11) **EP 2 310 085 B1**
(45) Veröffentlichungstag und Bekanntmachung des Hinweises auf die Patenterteilung: **02.09.2015**
(21) Anmeldenummer: 09753543.9
(22) Anmeldetag: 28.05.2009
(51) Int. Cl.: A61N 1/30, A61L 2/238

(54) **GERÄT ZUR REDUKTION PATHOGENER MIKROBEN**
DEVICE FOR REDUCING PATHOGENIC MICROBES
APPAREIL SERVANT À RÉDUIRE LES MICROBES PATHOGÈNES

(30) Priorität: 30.05.2008 DE 102008026067
(43) Veröffentlichungstag der Anmeldung: 20.04.2011
(73) Patentinhaber: Schaffrath, Paul, 55218 Ingelheim (DE)
(72) Erfinder: SCHAFFRATH, Paul, 55262 Heidesheim (DE); KUPPFER, Valerie, 55129 Mainz (DE)
(74) Vertreter: Kodron, Felix
(86) Internationale Anmeldenummer: PCT/DE2009/000743
(87) Internationale Veröffentlichungsnummer: WO 2009/143827

(56) Entgegenhaltungen:
- WO-A1-94/07550
- WO-A2-2005/062710
- US-A- 5 814 094

## Beschreibung

Die Erfindung betrifft ein Gerät zur Reduktion pathogener Mikroben.

Auf und im menschlichen Körper siedelt eine Vielzahl verschiedener Bakterien. Dabei ist die Mundhöhle eine der Haupteintrittspforten für pathogene Erreger wie Bakterien und Pilze.

Diese werden bislang im Falle einer Erkrankung z.B. mit Antibiotika und Antimykotika mehr oder weniger erfolgreich therapiert, wobei als unerwünschter Nebeneffekt stets Nebenwirkungen, Unverträglichkeiten und Resistenzen auftreten.

Bei mehr oder weniger allen Antibiotika sind u.a. gastrointestinale Störungen wie Übelkeit, Erbrechen, Durchfall sehr häufig. Auch allergische Reaktionen sind verbreitet und verbieten in vielen Fällen sogar eine antbiotische Behandlung des betroffenen Patienten.

Im menschlichen Mundraum sind beispielsweise zahlreiche Bakterienstämme anzutreffen, deren Stoffwechselund Zerfallsprodukte als sogenannte bakterielle Plaque (Zahnbelag) die Zähne überziehen. Durch die Anwesenheit der Plaque werden körpereigene Abwehrreaktionen gegen die im Mundraum siedelnden Bakterien ausgelöst, wobei u.a. Enzyme freigesetzt werden, die erfolglos versuchen, die Bakterien zu zerstören, und ein Vordringen der Mikroorganismen in tiefere Gewebeschichten zu verhindern, jedoch stattdessen zu einer Zerstörung des Eigengewebes führen. Die Bakterien sind hierbei durch einen Biofilm vor der Einwirkung von Antibiotika geschützt.

So kann es selbst bei täglich durchgeführter Mundhygiene durch die Nebeneffekte der körpereigenen Immunreaktion zu Entzündungen im Mundraum kommen, z.B. zu Zahnfleischbluten, Parodontitis, Knochenabbau etc., letztendlich zu irreversiblen Zerstörungen des Zahnhalteapparates.

Parodontitis, eine bakteriell bedingte Entzündung des Zahnhalteapparates mit allmählichem Abbau des Kieferknochens, ist eine Volkskrankheit, wobei etwa 80 % aller Deutschen von Parodontitis in unterschiedlichen Ausprägungsstufen betroffen sind.

Zur Parodontitistherapie wird üblicherweise eine gründliche Zahnreinigung empfohlen umfassend eine Zahnsteinund Plaqueentfernung. Da insbesondere die Ansammlung von Bakterien in sogenannten Zahnfleischtaschen problematisch ist, wird eine Parodontitisbehandlung regelmäßig unterstützt durch die Gabe von Antibiotika, die entweder lokal in die Zahnfleischtasche eingeführt oder systemisch, z.B. als Tabletten, verabreicht werden. Vorrangiges Ziel ist die rasche Reduzierung der Bakterienzahl im Mundraum des Patienten.

Im Mund kommt es aber auch durch andere pathogene Mikroben zu häufigen Krankheiten wie Herpes, Aphten oder Mykosen.

Stets ist eine medikamentöse Therapie die bislang am häufigsten angewandte Methode zur Heilung bei hoher Rezidivgefahr.

Ein anderes Beispiel ist die weite Verbreitung vaginaler Mykosen oder vaginaler bakterieller Infektionen. Auch hier kommen Antimykotika oder Antibiotika zum Einsatz mit den bereits geschilderten unerwünschten Folgen. Auch hier erschwert ein die Bakterien umgebender Biofilm die Einwirkung von Antibiotika.

Die nachteiligen Folgen regelmäßiger Antibiotikagaben sind hinlänglich bekannt; insbesondere die zunehmende Resistenz zahlreicher Bakterienstämme gegenüber häufig verwendeter Antibiotika ist hierbei sehr problematisch.

Eine antibakterielle Wirkung wird neben den bereits genannten Antibiotika auch z.B. durch Silberionen erreicht. So wurde Silber bereits in der Antike zur Wunddesinfektion oder zum Frischhalten von Wasser und Lebensmitteln verwendet.

Diese antibakterielle Wirkung des Silbers erklärt sich daraus, dass in feuchtem Milieu Silberionen freigesetzt werden, die die Zellwände von Bakterien in der näheren Umgebung angreifen und zerstören. Damit hat Silber eine antibakterielle Wirkung, die praktisch nebenwirkungsfrei zur Anwendung kommen kann.

Bekannt ist u.a. aus der Schrift DE 29803277 U1 die Verwendung von sogenannten Zungenreinigern aus Silber, die über die Zungenoberfläche gestrichen werden, wodurch die Zahl der auf der Zunge siedelnden Bakterien deutlich verringert wird.

Bekannt ist auch die Verwendung von Zahncremes bzw. Mundwässern und Zahnbürsten, die Silber enthalten. Beispielsweise in der DE 19508539 wird eine Zahnbürste mit Filamenten aus elastischem Kunststoff vorgeschlagen, in denen u.a. Silberpartikel enthalten sind, die unter dem Einfluss von Speichel Silber in ionischer Form freisetzen.

Dadurch wird erreicht, dass die Zahnbürste auch nach längerem Bürstengebrauch keine mikrobiellen Beläge aufweist.

Auch aus der DE 101 45 275 sind Kunststoffborsten, die Silberpartikel verschiedener Korngrößen beinhalten, zur Verwendung bei Zahnbürsten vorbekannt.

Nachteilig ist hierbei jedoch, dass die Herstellung solcher spezieller Zahnbürsten mit in die Filamente eingearbeiteten Silberpartikeln kostenintensiv ist.

Der Einsatz von Silberionen für antibakterielle Oberflächen in Kunststoffen ist neben der beschriebenen Verwendung bei Zahnbürsten auch z.B. bei bestimmten Lichtschaltern und Türgriffen in Kliniken, in Waschmaschinen, Wundverbänden oder als eingewebte Silberfäden in Sportbekleidung sowie u.a. auch als Bestandteil von Wandfarbe gegen Schimmelpilzbildung bekannt.

Die bisher angewandten Verfahren des Einbauens von Silberpartikeln in eine Polymermatrix ist jedoch technisch aufwändig und schwierig.

Bei allen vorgenannten Vorrichtungen und Verfahren wirken die Silberionen, die durch Feuchtigkeitskontakt in Lösung gehen, direkt an der Silber- bzw. Gewebeoberfläche. Jedoch ist eine Wirkung in tieferen Gewebeschichten so nicht zu erreichen.

Um allgemein eine Wirkung eines Medikaments nicht nur an der Gewebeoberfläche zu erzielen, sondern auch eine Tiefenwirkung im Gewebe zu erreichen, ist u.a. bekannt, den Wirkstofftransport in ein Gewebe mittels Ionophorese (= Iontophorese) zu erreichen.

Hierbei werden unter Einwirkung von schwachem Gleichstrom Wirkstoffe in einer Flüssigkeit gelöst in die Haut eingeschleust, wobei die Wirkstoffe als Ionen, d.h. als eigene Ladungsträger, vorliegen müssen, damit sie im elektrischen Feld transportiert werden können. Die Stromstärke ist dabei so niedrig, dass der Patient höchstens ein leichtes Kribbeln verspürt.

Bekannte Anwendungen der Ionophorese sind z.B. das Verbessern der Eindringtiefe antirheumatischer Salben in die Haut oder die Behandlung von überaktiven Schweißdrüsen durch Gleichstrom im Wasserbad.

Beispielsweise aus der WO 02/19941 A1 ist ein Ionophoresegerät bekannt, womit überempfindliches Dentin am Zahn behandelt wird durch das Aufbringen spezieller Flüssigkeiten.

Der Patient hält dabei ein in etwa stabförmiges Gerät in seiner Hand, welches in seinem Inneren eine Stromquelle sowie eine mit medizinischer Wirkflüssigkeit gefüllte Patrone besitzt, die über ein Kopfstück auf die Zahnoberfläche im Mund aufgebracht wird. Sobald das Kopfstück den Zahn berührt, wird über den Arm des Patienten der Stromkreis geschlossen und der Wirkstoff in den Zahn eingeschleust.

In der US 3520279 A ist eine ionophoretisch wirkende Zahnbürste beschrieben, die die unterschiedliche Leitfähigkeit verschiedener Metalle in ihrem Kopfbereich ausnutzt, um Wirkstoffe der Zahnpasta in das Zahninnere zu transportieren. Als bekannt guter elektrischer Leiter wird hierbei auch das Material Silber erwähnt.

Um grundsätzlich den Wirkstoff per Ionophorese möglichst nah an den Krankheitsherd heranzubringen, sind auch spezielle Formkörper an Geräten bekannt, die insbesondere im Mundraum zur Behandlung von Parodontitis verwendet werden.

Beispielhaft sei hier auf die US Patentschrift Nr. 3502076 A verwiesen, in welcher ein speziell abdrucklöffelförmiges Mundstück mit einem Schwamm offenbart ist, über welches mittels Ionophorese flüssige Medikamente in das Innere von Zähnen und Kiefer transportiert werden. Der Stromkreis wird hierbei über den Körper des Patienten geschlossen.

In der DE 2839968 C2 ist ebenfalls eine speziell geformte u-förmige Schiene im Mundraum offenbart, die an ihren gegenüberliegenden Schenkeln beide Elektroden trägt, so dass der Strom nur im Mund, und nicht durch andere Körperteile fließt. Weiterhin lässt sich die Stromflussrichtung umpolen. In der Schiene ist wiederum ein flüssiger Wirkstoff untergebracht, so dass auch dieses Gerät dem Wirkstofftransport in tiefere Gewebeschichten durch Ionophorese dient. Es wird vorgeschlagen, u.a. Silber als Elektroden zu verwenden aufgrund seiner bekannten guten Leitfähigkeit.

Nachteilig bei den bekannten Ionophoresegeräten ist, dass stets ein Medikament in das erkrankte Gewebe eingeschleust wird, d.h. es erfolgt eine Behandlung im Krankheitsfall mit einem medizinischen Wirkstoff, auf den der menschliche Körper bei Anwendung über einen längeren Zeitraum zumeist mit Nebenwirkungen oder Resistenzen reagiert. Ein möglicherweise antimikrobieller Effekt der verwendeten Elektroden spielt bei der Ionophorese kein Rolle. Hier ist lediglich eine gute Leitfähigkeit wichtig, die z.B. auch das Metall Silber besitzt.

Aus der US 5,814,094 ist ein ionophoretisch wirkendes System zur Wundheilung bekannt, bei welcher ein silberhaltiges Wundpflaster auf ein verletztes Hautareal aufgebracht wird.

Das Pflaster wirkt dabei als Anode; auf einem intakten Hautareal in Wundnähe wird eine Kathode fixiert und zwischen den beiden Polen in bekannter Weise mittels eines Spannungsgerätes eine Niedrigspannung angelegt, so dass Silberionen aus dem Pflaster in die Wunde einwandern um dort während einer tagelangen, ununterbrochenen Behandlung unter ärtztlicher Aufsicht vorallem einen Silber-Kollagen-Komplex zu bilden, wodurch die Zellteilung stimuliert werden soll.

Es wird in dieser Schrift ebenfalls erwähnt, dass Silberionen einen antimikrobiellen Effekt aufweisen, jedoch ist das offenbarte System für den Einsatz im Krankenhaus über mehrere Tage bis zur abschließenden Wundheilung vorgesehen.

Zusammenfassend ist festzustellen, dass die bisher bekannten und angewendeten mechanischen Methoden bzw. medikamentösen Therapien nicht ausreichen, um eine wirklich dauerhafte Verringerung der Zahl krankheitserregener Mikroben auf und im Körper zu erreichen, wodurch sich auch die das Eigengewebe schädigende körpereigene Immunabwehr abschwächen ließe.

Es ist daher die Aufgabe der vorliegenden Erfindung, ein einfach zu bedienendes Gerät zur Reduktion pathogener Mikroben unter Ausnutzung des antimikrobiellen Effektes von Silberionen ohne zusätzliche medikamentöse Behandlung zu schaffen, so dass es praktisch jedem Menschen möglich ist, eine wirkungsvolle Behandlung am eigenen Körper als Prophylaxe, z.B. Parodontitisprophylaxe bei der täglichen Zahnpflege, oder aber im Krankheitsfall durchzuführen, ohne fortlaufend ein Medikament einnehmen oder anwenden zu müssen.

Erreicht wird dies nach der Erfindung durch ein Gerät mit den Merkmalen des Anspruchs 1.

Vorteilhafte Ausführungsformen sind in den Unteransprüchen ausgeführt.

In den Figuren sind vorteilhafte Bauformen der Erfindung näher erläutert und beschrieben.

Es zeigen
- Figur 1: das erfindungsgemäße Gerät schematisiert etwa in natürlicher Größe mit einer Silberschicht am Pluspol am Gerätekopf und
- Figur 2: ein als Vaginalsonde ausgebildetes spezielles Kopfteil des erfindungsgemäßen Gerätes.

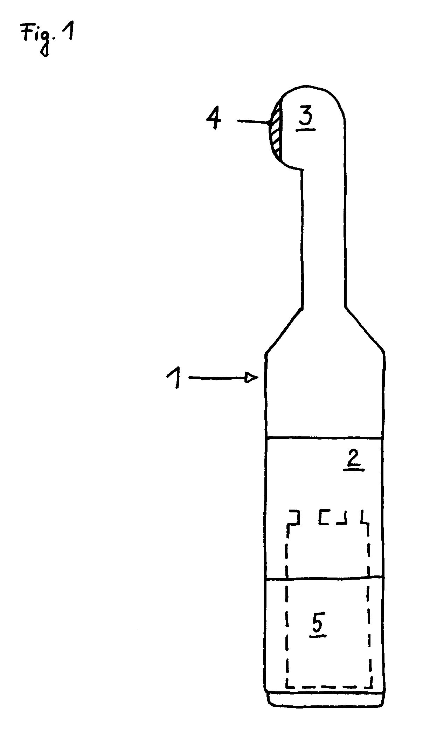

Das in Figur 1 dargestellte erfindungsgemäße Gerät 1 besteht aus einem Gehäuse 2 aus sterilisierbarem Kunststoff oder Edelstahl, einer internen Stromquelle 5 und einem auswechselbaren Kunststoffkopf 3 mit einer vorzugsweise runden oder elliptischen Silberelektrode 4 als Pluspol. Das Gerät 1 soll beispielsweise vom Patienten in die rechte Hand genommen werden, wobei die Hand den leitfähigen Mittelbereich (Minuspol) 6 des Gehäuses 2 fest umschließt.

Dann führt der Patient den Gerätekopf 3 in seinen Mundraum ein und beginnt, sein Zahnfleisch, insbesondere die Zahnfleischsäume an den Zähnen, gleichmäßig von innen und außen sowie oben und unten zu massieren, wobei die als Pluspol wirkende Silberelektrode 4 mit der leitfähigen Silberfolie direkt auf dem Zahnfleisch zu liegen kommt.

Durch den Kontakt mit dem feuchten Zahnfleisch fließt durch den Arm des Patienten ein nicht oder kaum wahrnehmbarer Niedrigstrom etwa im Bereich von 0,5 mA aus einer im Geräteinneren untergebrachten Batterie 5, d.h. der Stromkreis wird geschlossen, und es werden am Pluspol 4 am Gerätekopf 3 Silberionen freigesetzt. Genauso können die Zunge oder die Wangenschleimhaut behandelt werden.

Eine nicht dargestellte Regelelektronik sorgt für gleichbleibenden Stromfluss und verhindert eine Überspannung, z.B. bei Kontakt mit einer metallischen Restauration im Mundraum.

Es besteht auch die Möglichkeit, die Stromstärke über einen Regler 7 zu regulieren.

Die freigesetzten Ag+-Ionen der Silberelektrode 4 am Gerätekopf 3 binden sich an die äußere Zellwand der im Mundraum befindlichen Bakterien und schädigen die Bakterienzellen irreversibel. Weitere, bei der klassischen Ionophorese stets verwendete Medikamente mit Nebenwirkungen, kommen bei diesem erfinderischen Gerät nicht zum Einsatz.

Durch die Wirkungsweise des erfindungsgemäßen Gerätes ist eine regelmäßige und dauerhafte Eliminierung der pathogenen Bakterien, die z.B. für die Parodontitis verantwortlich sind, möglich, und zwar ohne allergische Reaktionen, Resistenzentwicklungen oder die bekannten Nebenwirkungen von Antibiotika.

Diese wirkungsvolle Bakterieneliminierung kann von jedem Patienten problemlos durch Integration in die häusliche Mundhygiene durchgeführt werden ebenso wie im Rahmen der professionellen Zahnreinigung beim Zahnarzt. Es sind daher sowohl einfache Ausführungen des Gerätes für die Heimanwendung mit integrierter Spannungsquelle wie bei elektrischen Zahnbürsten möglich, als auch Geräte für die professionelle Verwendung durch Zahnärzte und Prophylaxeassistenten, die ggf. eine externe Spannungsversorgung und weitere bauliche Besonderheiten wie z.B. die Auslagerung des Minuspols als Metallteil, welches der Patient in die Hand nimmt, verbunden mit dem Gerät mit einem isoliertem Kabel sowie auswechselbare und sterilisierbare Gerätekopfstücke verschiedener Formen und Größen vorgesehen, die für den professionellen Einsatz des Gerätes erforderlich sind.

Die Silberschicht zumindest am Pluspol kann als dickere Silberschicht oder aber als auswechselbare dünnere Silberfolie ausgebildet sein für den Einmalgebrauch.

Aufgrund der Tatsache, dass Silberionen bereits bei geringsten Stromstärken hoch antimikrobiell wirken, d.h. nicht nur gegenüber Bakterien, sondern auch gegen Pilze und Viren effektiv angewendet werden können, kann das erfindungsgemäße Gerät praktisch an jeder Stelle im und am menschlichen Körper zum Einsatz kommen, sofern zumindest der silberne Pluspol möglichst paßgenau an die zu behandelnde Körperstelle gebracht wird, so dass für jede Anwendung das Kopfstück speziell geformt sein muss.

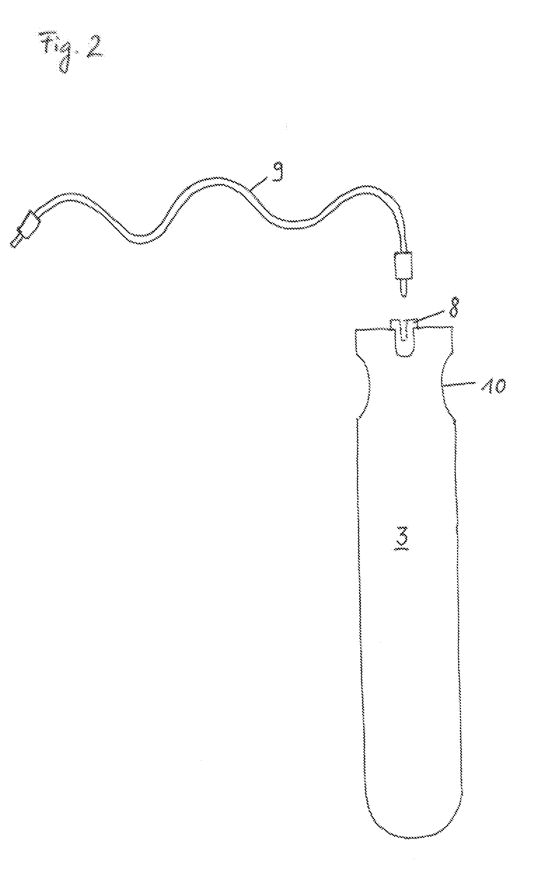

Figur 2 zeigt ein spezielles, als in etwa zylinderförmige Vaginalsonde ausgebildetes Kopfteil 3 des erfindungsgemäßen Gerätes 1, welches über eine Buchse 8 und ein Kabel 9 mit dem Handgriff (Minuspol) des Gerätes 1 verbunden ist. An ihrem einen Ende ist die Vaginalsonde 3 als weiterer Griff 10 ausgeformt.

Dieses spezielle Kopfteil 3 kann in verschiedenen Größen ausgebildet sein und ist vorteilhafterweise sterilisierbar, so dass es mehrfach angewendet werden kann. Wesentlich ist, dass das Kopfteil 3 entweder ganz oder teilweise aus Silber besteht, welches mit der inneren Schleimhaut in Berührung kommt und bei Stromfluß antimikrobiell wirksame Silberionen abgibt.

Grundsätzlich werden in der Medizin ähnlich geformte Vaginalsonden ohne Silber mit Reizstrom eingesetzt, z.B. zur Stärkung der Beckenbodenmuskulatur, jedoch ist der Einsatz von antimikrobiell wirkenden Silberionen dabei nicht bekannt.

Da Silberionen auch bestimmte Krebszellen an der Teilung hindern, ist auch ein Einsatz in diese Richtung möglich, z.B. bei Gebärmutterhalskrebs, oder durch eine in der Form entsprechend angepasste Rektalsonde bei Darm- und Prostatakrebs.

Es ist aus zahlreichen Untersuchungen erwiesen, dass Silberionen gegen alle bekannten pathogenen Bakterien sowie auch Pilze und Viren effektiv wirken, ohne dass diese Resistenzen gegen Silberionen entwickeln können. Unverträglichkeiten oder Allergien gegen Silber sind nicht bekannt. Als bislang einzige Nebenwirkung ist bei zu hoher Silberaufnahme eine graue Verfärbung der Haut oder Schleimhaut beschrieben (Argyrie), die z.B. nach der Einnahme silberhaltiger Medikamente oder bei der Verwendung silberhaltiger Schminke beobachtet wurde. Die Graufärbung ist bedingt durch die Einlagerung silberfarbener Granuli in die Haut. Als Grenzwert für Argyrie gilt die intravenöse Aufnahme von 1 mg Silber pro Tag (Erwachsener, 70 kg).

Durch das erfindungsgemäße Gerät wird eine derart hohe Silberaufnahme in den Körper, wie sie für das Auftreten der Argyrie nötig wäre, in keiner Weise erreicht.

Durch die Wirksamkeit des erfindungsgemäßen Gerätes auf pathogene Mikroben ohne die bekannten Nachteile wie Nebenwirkungen, Allergien, Resistenzen, Anwendungsbeschränkungen und Unverträglichkeiten, die bei der Einnahme oder Anwendung von Medikamenten unvermeidbar auftreten, erhält der Anwender die Möglichkeit einer effektiven, einfachen und kostengünstigen Prophylaxe und Therapie mit langfristigem Erfolg ohne gesundheitliche Belastung des eigenen Körpers.

## Patentansprüche

1. Gerät (1) zur Reduktion pathogener Mikroben, welches als Handgerät ausgebildet ist, umfassend einen Handgriff (2), einen mit dem Körper in Kontakt tretenden Minuspol sowie einen Gerätekopf (3) mit einem Pluspol (4), wobei zwischen den Polen eine elektrische Niedrigspannung am Handgerät erzeugbar ist, **dadurch gekennzeichnet, dass** der Pluspol (4) am Gerätekopf (3) als runder oder elliptischer oder stabförmiger Körper aus Silber ausgebildet ist, welcher konfiguriert ist beim Schließen des Stromkreises positive Ionen in das Körpergewebe abzugeben, und der Minuspol im Handgriff (2) des Handgerätes (1) in dessen Mittelbereich als leitfähige Zone integriert ist.

2. Handgerät (1) zur Reduktion pathogener Mikroben im Bereich der Schleimhäute nach Anspruch 1,
**dadurch gekennzeichnet, dass**
zumindest der Pluspol aus 999er Feinsilber besteht.

3. Handgerät (1) zur Reduktion pathogener Mikroben im Bereich der Schleimhäute nach einem der vorangegangenen Ansprüche,
**dadurch gekennzeichnet, dass**
zumindest der Pluspol aus einer auswechselbaren Silberfolie besteht.

4. Handgerät (1) zur Reduktion pathogener Mikroben im Bereich der Schleimhäute nach einem der vorangegangenen Ansprüche,
**dadurch gekennzeichnet, dass**
im Handgeräteinneren eine Stromquelle (5) untergebracht ist.

5. Handgerät (1) zur Reduktion pathogener Mikroben im Bereich der Schleimhäute nach einem der vorangegangenen Ansprüche,
**dadurch gekennzeichnet, dass**
der Gerätekopf (3) des Handgerätes (1) abnehmbar und austauschbar ist, wobei der Gerätekopf (3) über eine Metallsteckverbindung am Handgerät (1) befestigt ist.

6. Handgerät (1) zur Reduktion pathogener Mikroben im Bereich der Schleimhäute nach einem der vorangegangenen Ansprüche,
**dadurch gekennzeichnet, dass**
der Gerätekopf (3) des Handgeräts (1) über Buchsen (8) und Kabel (9) mit dem Handgriff (2) verbunden ist und so vom Handgriff (2) entfernt angewendet werden kann.

7. Handgerät (1) zur Reduktion pathogener Mikroben im Bereich der Schleimhäute nach einem der vorangegangenen Ansprüche,
**dadurch gekennzeichnet, dass**
am Handgerät (1) zwei Buchsen angeordnet sind, von denen eine mit der Kathode der innenliegenden Spannungsquelle und die andere mit der leitfähigen Zone am Handgriff (2) verbunden ist.

8. Handgerät (1) zur Reduktion pathogener Mikroben im Bereich der Schleimhäute nach Anspruch 7,
**dadurch gekennzeichnet, dass**
die beiden Buchsen am Handgerät (1) bei der eigenhändigen Anwendung durch eine Person elektrisch überbrückt werden, wodurch bei Berührung der leitfähigen Zone am Handgriff (2) der Stromkreis über den Arm der das Handgerät (1) benutzenden Person geschlossen wird, sobald der Gerätekopf (3) des Handgerätes (1) Kontakt mit dessen Körper bekommt.

9. Handgerät (1) zur Reduktion pathogener Mikroben im Bereich der Schleimhäute nach einem der vorangegangenen Ansprüche 1 bis 7,
**dadurch gekennzeichnet, dass**
der Gerätekopf (3) des Handgerätes (1) mit zumindest dem Pluspol (4) aus Silber in etwa stabförmig ausgebildet ist.

10. Handgerät (1) zur Reduktion pathogener Mikroben im Bereich der Schleimhäute nach Anspruch 09,
**dadurch gekennzeichnet, dass**
der in etwa stabförmige Gerätekopf (3) abnehmbar und austauschbar ist und über zumindest eine Buchse (8) und zumindest ein Kabel (9) mit dem übrigen Handgerät (1) verbunden ist, wobei an einem Ende des in etwa stabförmigen Gerätekopfs (3) ein weiterer Griff (10) angeordnet ist.

11. Handgerät (1) zur Reduktion pathogener Mikroben im Bereich der Schleimhäute nach einem der vorangegangenen Ansprüche,
**dadurch gekennzeichnet, dass**
zumindest der Gerätekopf (3) des Handgerätes (1) aus sterilisierbarem Material besteht.

12. Handgerät (1) zur Reduktion pathogener Mikroben im Bereich der Schleimhäute nach einem der vorangegangenen Ansprüche,
**dadurch gekennzeichnet, dass**
die Spannungsquelle (5) wiederaufladbar ist und eine separate Ladestation besitzt.

13. Handgerät (1) zur Reduktion pathogener Mikroben im Bereich der Schleimhäute nach einem der vorangegangenen Ansprüche,
**dadurch gekennzeichnet, dass**
das Handgerät(1) eine Regelelektronik zur Vermeidung einer Überspannung bei Kontakt mit metallischen Restaurationen im und am Körper besitzt.

14. Handgerät (1) zur Reduktion pathogener Mikroben im Bereich der Schleimhäute nach einem der vorangegangenen Ansprüche,
**dadurch gekennzeichnet, dass**
am Handgerät (1) eine Zeitmessvorrichtung angeordnet ist.

15. Handgerät (1) zur Reduktion pathogener Mikroben im Bereich der Schleimhäute nach einem der vorangegangenen Ansprüche,
**dadurch gekennzeichnet, dass**
am Handgerät (1) ein Potentiometer (7) zur Regulierung der Spannung angeordnet ist, und dass
das Handgerät (1) über eine externe regelbare Spannungsquelle mit Spannung versorgt wird.

## Claims

1. Device (1) for reducing pathogenic microbes, which device is in the form of a hand-held device, comprising a hand grip (2), a negative terminal coming into contact with the body, and also a device head (3) with a positive terminal (4), wherein a low electrical voltage can be generated in the hand-held device between the terminals,
**characterized in that**
the positive terminal (4) on the device head (3) is in the form of a round or elliptical or rod-shaped body composed of silver which is configured to release positive ions into the body tissue upon closing the electric circuit, and the negative terminal in the hand grip (2) of the hand-held device (1) is integrated as a conductive zone in the midsection of said hand grip.

2. Hand-held device (1) for reducing pathogenic microbes in the region of the mucosae according to Claim 1,
**characterized in that**
at least the positive terminal consists of 0.999 fine silver.

3. Hand-held device (1) for reducing pathogenic microbes in the region of the mucosae according to either of the preceding claims,
**characterized in that**
at least the positive terminal consists of a replaceable silver foil.

4. Hand-held device (1) for reducing pathogenic microbes in the region of the mucosae according to any of the preceding claims,
**characterized in that**
a current source (5) is accommodated within the hand-held device.

5. Hand-held device (1) for reducing pathogenic microbes in the region of the mucosae according to any of the preceding claims,
**characterized in that**
the device head (3) of the hand-held device (1) is detachable and replaceable, wherein the device head (3) is attached to the hand-hald device (1) via a metal plug connection.

6. Hand-held device (1) for reducing pathogenic microbes in the region of the mucosae according to any of the preceding claims,
**characterized in that**
the device head (3) of the hand-held device (1) is connected to the hand grip (2) via sockets (8) and cables (9) and, thus, can be applied away from the hand grip (2).

7. Hand-held device (1) for reducing pathogenic microbes in the region of the mucosae according to any of the preceding claims,
**characterized in that**
there is an arrangement on the hand-held device (1) of two sockets, one of which is connected to the cathode of the interior voltage source and the other one is connected to the conductive zone in the hand grip (2).

8. Hand-held device (1) for reducing pathogenic microbes in the region of the mucosae according to claim 7,
**characterized in that**
both sockets on the hand-held device (1) are bridged electrically by a person during use with his/her own hand, whereby, upon touching the conductive zone in the hand grip (2), the electric circuit is closed via the arm of the person using the hand-held device (1) as soon as the device head (3) of the hand-held device (1) gets into contact with the body of said person.

9. Hand-held device (1) for reducing pathogenic microbes in the region of the mucosae according to any of the preceding Claims 1 to 7,
**characterized in that**
the device head (3) of the hand-held device (1) with at least the positive terminal (4) made of silver is more or less rod-shaped.

10. Hand-held device (1) for reducing pathogenic microbes in the region of the mucosae according to Claim 9,
**characterized in that**
the more or less rod-shaped device head (3) is detachable and replaceable and is connected to the rest of the hand-held device (1) via at least one socket (8) and at least one cable (9), wherein a further grip (10) is arranged at one end of the more or less rod-shaped device head (3).

11. Hand-held device (1) for reducing pathogenic microbes in the region of the mucosae according to any of the preceding claims,
**characterized in that**
at least the device head (3) of the hand-heed device (1) consists of sterilizable material.

12. Hand-held device (1) for reducing pathogenic microbes in the region of the mucosae according to any of the preceding claims,
**characterized in that**
the voltage source (5) is rechargeable and has a separate charging station.

13. Hand-held device (1) for reducing pathogenic microbes in the region of the mucosae according to any of the preceding claims,
**characterized in that**
the hand-held device (1) has an electronic control system for avoiding overvoltage upon contact with metal restorations in and on the body.

14. Hand-held device (1) for reducing pathogenic microbes in the region of the mucosae according to any of the preceding claims,
**characterized in that**
a timepiece is arranged on the hand-held device (1).

15. Hand-held device (1) for reducing pathogenic microbes in the region of the mucosae according to any of the preceding claims,
**characterized in that**
a potentiometer (7) for controlling voltage is arranged on the hand-held device (1), and **in that**
the hand-held device (1) is provided with voltage via an external controllable voltage source.

## Revendications

1. Appareil (1) de réduction des microbes pathogènes, lequel est réalisé sous la forme d'un appareil portatif, comprenant une poignée (2), un pôle négatif qui entre en contact avec le corps ainsi qu'une tête d'appareil (3) munie d'un pôle positif (4), une basse tension électrique pouvant être générée sur l'appareil portatif entre les pôles,
**caractérisé en ce que**
le pôle positif (4) sur la tête d'appareil (3) est réalisé sous la forme d'un corps rond ou elliptique ou en forme de barre en argent, lequel est configuré pour délivrer des ions positifs dans le tissu corporel lors de la fermeture du circuit électrique, et le pôle négatif est intégré dans la poignée (2) de l'appareil portatif (1) sous la forme d'une zone conductrice dans sa zone centrale.

2. Appareil portatif (1) de réduction des microbes pathogènes dans la zone des muqueuses selon la revendication 1, **caractérisé en ce qu'**au moins le pôle positif se compose d'argent fin pur à 999 %

3. Appareil portatif (1) de réduction des microbes pathogènes dans la zone des muqueuses selon l'une des revendications précédentes, **caractérisé en ce qu'**au moins le pôle positif se compose d'un film en argent interchangeable.

4. Appareil portatif (1) de réduction des microbes pathogènes dans la zone des muqueuses selon l'une des revendications précédentes, **caractérisé en ce qu'**une source de courant (5) est logée à l'intérieur de l'appareil portatif.

5. Appareil portatif (1) de réduction des microbes pathogènes dans la zone des muqueuses selon l'une des revendications précédentes, **caractérisé en ce que** la tête d'appareil (3) de l'appareil portatif (1) est amovible et remplaçable, la tête d'appareil (3) étant fixée à l'appareil portatif (1) par le biais d'une liaison à enficher métallique.

6. Appareil portatif (1) de réduction des microbes pathogènes dans la zone des muqueuses selon l'une des revendications précédentes, **caractérisé en ce que** la tête d'appareil (3) de l'appareil portatif (1) est reliée à la poignée (2) par des douilles (8) et des câbles (9) et elle peut ainsi être utilisée en étant retirée de la poignée (2).

7. Appareil portatif (1) de réduction des microbes pathogènes dans la zone des muqueuses selon l'une des revendications précédentes, **caractérisé en ce que** deux douilles sont disposées sur l'appareil portatif (1), dont l'une est reliée à la cathode de la source de tension interne et l'autre à la zone conductrice sur la poignée (2).

8. Appareil portatif (1) de réduction des microbes pathogènes dans la zone des muqueuses selon la revendication 7, **caractérisé en ce que** les deux douilles sur l'appareil portatif (1) sont court-circuitées électriquement lors de l'utilisation en mains propres par une personne, le circuit électrique étant ainsi fermé par le bras de la personne qui utilise l'appareil portatif (1) lors du contact de la zone conductrice sur la poignée (2) dès que la tête d'appareil (3) de l'appareil portatif (1) entre en contact avec son corps.

9. Appareil portatif (1) de réduction des microbes pathogènes dans la zone des muqueuses selon l'une des revendications précédentes 1 à 7, **caractérisé en ce que** la tête d'appareil (3) de l'appareil portatif (1) avec au moins le pôle positif (4) en argent est configurée approximativement en forme de barre.

10. Appareil portatif (1) de réduction des microbes pathogènes dans la zone des muqueuses selon la revendication 9, **caractérisé en ce que** la tête d'appareil (3) approximativement en forme de barre est amovible et remplaçable et elle est reliée au reste de l'appareil portatif (1) par au moins une douille (8) et au moins un câble (9), une poignée supplémentaire (10) étant disposée à une extrémité de la tête d'appareil (3) approximativement en forme de barre.

11. Appareil portatif (1) de réduction des microbes pathogènes dans la zone des muqueuses selon l'une des revendications précédentes, **caractérisé en ce qu'**au moins la tête d'appareil (3) de l'appareil portatif (1) se compose d'un matériau pouvant être stérilisé.

12. Appareil portatif (1) de réduction des microbes pathogènes dans la zone des muqueuses selon l'une des revendications précédentes, **caractérisé en ce que** la source de tension (5) est rechargeable et possède une station de charge séparée.

13. Appareil portatif (1) de réduction des microbes pathogènes dans la zone des muqueuses selon l'une des revendications précédentes, **caractérisé en ce que** l'appareil portatif (1) possède une électronique de régulation destinée à éviter une surtension en cas de contact avec des prothèses métalliques dans et sur le corps.

14. Appareil portatif (1) de réduction des microbes pathogènes dans la zone des muqueuses selon l'une des revendications précédentes, **caractérisé en ce qu'**un dispositif de mesure du temps est disposé sur l'appareil portatif (1).

15. Appareil portatif (1) de réduction des microbes pathogènes dans la zone des muqueuses selon l'une des revendications précédentes, **caractérisé en ce qu'**un potentiomètre (7) servant à la régulation de la tension est disposé sur l'appareil portatif (1) et **en ce que** l'appareil portatif (1) est alimenté en tension par le biais d'une source de tension réglable externe.
